# EUROPÄISCHE PATENTSCHRIFT

(11) **EP 0 330 995 B1**
(45) Veröffentlichungstag und Bekanntmachung des Hinweises auf die Patenterteilung: **31.08.1994**
(21) Anmeldenummer: 89103137.9
(22) Anmeldetag: 23.02.1989
(51) Int. Cl.: C07C 43/188, C07C 43/303, C07C 403/00, C11B 9/00

(54) **Bicyclische Aether**
Bicyclic ethers
Ethers bicycliques

(30) Priorität: 04.03.1988 CH 819/88
(43) Veröffentlichungstag der Anmeldung: 06.09.1989
(73) Patentinhaber: GIVAUDAN-ROURE (INTERNATIONAL) S.A., CH-1214 Vernier, Genève (CH)
(72) Erfinder: Fráter, Georg, Dr., CH-8610 Uster (CH); Schmidt, Harald, Dr., CH-8607 Aathal-Seegräben (CH)
(74) Vertreter: Urech, Peter, Dr.

(56) Entgegenhaltungen:
- US-A- 2 803 662
- HELVETICA CHIMICA ACTA XXXVIII, Fasc. VI, 15. Oktober 1955, Seiten 1573-1587;M. STOLL et al.: "190. Odeur et constitution XIII. Synthèse du triméthyl-1,1,5-méthylène-9-bicyclo-[3,3,1]-nonanol-5"

## Beschreibung

Die Erfindung betrifft neue Riechstoffgemische, nämlich Gemische von Verbindungen der Formeln 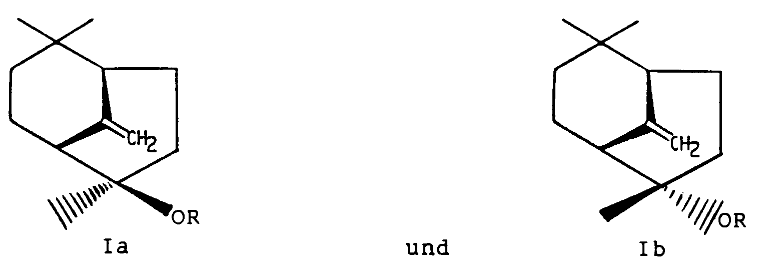
worin R = C₁₋₄-Alkyl, z.B. Methyl, Aethyl, Propyl, Isopropyl, Butyl, bedeutet,
in einem Verhältnis von mindestens 1,1:1, insbesondere von 2,2-3,0:1.

Ia wird in der Folge auch als exo-Form, Ib als endo-Form bezeichnet. Die erfindungsgemässen Gemische werden in der Folge auch mit "I" bezeichnet.

Die Erfindung betrifft ferner ein Verfahren zur Herstellung der erfindungsgemässen Gemische I.

Dieses Verfahren ist dadurch gekennzeichnet, dass man ein Dihydro-α-ionon-di-C₁₋₄-alkylketal oder einen C₁₋₄-Alkylenoläther von Dihydro-α-ionon mittels einer starken Säure cyclisiert.

Als starke Säuren kommen Protonsäuren oder Lewissäuren in Frage. Beispiele sind: Chlorwasserstoffgas, Schwefelsäure, Phosphorsäure, Trifluoressigsäure, Methansulfonsäure, p-Toluolsulfonsäure, Camphersulfonsäure, BF₃, BF₃·Aet₂O, BF₃·H₃PO₄, saure Ionentauscher, z.B. Dowex 50, Nafion u.a., etc.

Die genannten Ausgangsmaterialien stellt man vorzugsweise in situ her; dies zweckmässigerweise durch Umsetzung von Dihydro-α-ionon mit einem entsprechenden Ketalisierungsmittel. Geeignete Ketalisierungsmittel sind insbesondere die Ortho-ester HC(OR)₃. Diese Ester setzt man zweckmässigerweise mit Dihydro-α-ionon in Anwesenheit einer der oben genannten Säuren bei ca. 40°C bis ca. 50°C um.

Geeignete Menge Säure sind sowohl katalytische Mengen, aber auch grössere Mengen, z.B. bis ca. 10 Mol-%.

Man arbeitet gegebenenfalls in einem Lösungsmittel, z.B. einem - gegebenenfalls halogenierten - Kohlenwasserstoff. Zweckmässige Lösungsmittel sind demzufolge: Hexan, Cyclohexan, Methylenchlorid, Aethylenchlorid, Chloroform, Alkohol, etc.

Bei dieser Verfahrensweise werden die Stufen Ketal und Enoläther durchlaufen. Da insbesondere der Enoläther schon unter Einfluss von katalytischen Mengen Säure spontan cyclisiert, kann bei dessen Verwendung als Ausgangsmaterial schon bei tiefen Temperaturen, d.h. ca. -10°C bis +10°C, insbesondere 0°C bis 10°C gearbeitet werden. Obschon katalytische Mengen Säure genügen, kommen aber auch höhere Mengen, z.B. bis ca. 0,15 Mol/Mol Edukt in Frage.

Zwecks Aufarbeitung wird das Reaktionsprodukt zweckmässigerweise mit Base neutralisiert, und es kann hierauf zwecks Isolation destilliert werden.

Die Gemische I zeichnen sich insbesondere durch kräftige und sehr natürlich-warme Noten in Richtung Tabak, Ambergris und Holz aus. In Kompositionen kommt insbesondere diese letztere Holznote überwiegend zum Tragen, wobei gleichzeitig ausgesprochen harmonisierende Effekte beobachtet werden, wie diese üblicherweise nur mit Zusätzen - komplex zusammengesetzter - ätherischer Oele erzielt werden können. Die Kompositionen zeichnen sich überdies durch eine gesteigerte Diffusion und stark verbesserte Substantivität aus.

Es wurde nun überraschenderweise gefunden, dass es gerade der höhere Gehalt an exo-Isomerem Ia ist, der für die interessanten organoleptischen Effekte der erfindungsgemäsden Gemische verantwortlich ist, und welcher höhere Gehalt an exo-Isomerem durch das erfindungsgemässe Verfahren zur Verfügung gestellt wird. Ein möglichst hoher Gehalt an Ia wird z.B. dadurch erzielt, dass man auf der Stufe des Enoläthers, zweckmässigerweise wie oben beschrieben, isoliert.

Im Vordergrund des Interesses steht der Aethyläther, er besitzt einen holzigen, ambraartigen, blumigen Geruch (Richtung Iriswurzel) mit Aspekten von Patchouli, Muskatellersalbei und Tabak.

Der Methyläther weist ähnliche Geruchseigenschaften auf.

Die Erfindung betrifft demgemäss auch die Verwendung der Gemische I als Riechstoffe.

Die Gemische der Formel I eignen sich aufgrund ihrer natürlichen Geruchsnoten insbesondere zur Modifizierung von bekannten Kompositionen.

Bemerkenswert ist die Art und Weise, wie die Gemische I die Geruchsnoten bekannter Kompositionen abrunden und harmonisieren, ohne aber in unangenehmer Weise zu dominieren.

Die Gemische I verbinden sich mit zahlreichen bekannten Riechstoffingredientien natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist:
- Naturprodukte, wie Baummoos-Absolue, Basilikumöl, Agrumenöle (wie Bergamotteöl, Mandarinenöl, etc.), Mastix-Absolue, Myrtenöl, Palmarosaöl, Patchouliöl, Petitgrainöl Paraguay, Wermutöl,
- Alkohole, wie Farnesol, Geraniol, Linalool, Nerol, Phenyläthylalkohol, Rhodinol, Zimtalkohol,
- Aldehyde, wie Citral, Helional®, α-Hexylzimtaldehyd, Hydroxycitronellal, Lilial® (p-tert.Butyl-α-methyl-dihydrozimtaldehyd), Methylnonylacetaldehyd,
- Ketone, wie Allyljonon, α-Jonon, β-Jonon, Isoraldein (Isomethyl-α-ionon), Methyljonon,
- Ester, wie Allyl-phenoxyacetat, Benzyl-salicylat, Cinnamylpropionat, Citronellyl-acetat, Citronellyl-äthoxolat (Citronellyl . O - CO -CO . OC₂H₅), Decylacetat, Dimethylbenzylcarbinyl-acetat, Dimethylbenzylcarbinyl-butyrat, Aethyl-acetoacetat, Aethyl-acetylacetat, Hexenyl-isobutyrat, Linalylacetat, Methyl-dihydrojasmonat, Styrallylacetat, Vetiverylacetat, etc.
- Lactone, wie γ-Undecalacton,
- verschiedene, in der Parfümerie oft benützte Komponenten, wie Ketonmoschus, Indol, p-Menthan-8-thiol-3-on, Methyleugenol.

Die Gemische I lassen sich in weiten Grenzen einsetzen, die beispielsweise von ca. 0,1 (Detergentien) ca. -20% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 1 und ca. 10%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Gewebeveredler, Tabak, etc.).

Die Gemische I können demgemäss bei der Herstellung von Kompositionen und - wie obige Zusammenstellung zeigt - unter Verwendung einer breiten Palette bekannter Riechstoffe bzw. Riechstoffgemische verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1974 hervorgehend.

### Beispiel 1

### 2-Methoxy-9-methylen-2,6,6-trimethyl-bicyclo[3.3.1]nonan

120 g (0,563 Mol) Dihydro-α-ionon, 65 g (0,612 Mol) Orthoameisensäure-trimethylester und 200 ml Methylenchlorid werden unter Rühren während 15 Minuten tropfenweise mit einer Lösung von 8 g Bortrifluorid-ätherat in 60 ml Methylenchlorid versetzt und anschliessend während 105 Minuten unter Rückfluss gehalten. Das Reaktionsgemisch wird gekühlt und in 500 ml einer 5%igen wässrigen Kaliumhydroxid-lösung gegossen. Nach Zugabe von 500 ml Methylenchlorid und kräftiger Durchmischung werden die Phasen getrennt, die organische Phase mit Wasser gewaschen und das Lösungsmittel wird abdestilliert. Der Rückstand (197,1 g) wird im Vakuum über eine 15 cm Widmer-Kolonne fraktioniert. Es werden 100,2 g Produkt von Sdp. 44-47°/0,06-0,07 mbar (Ausbeute 87% d.Th. bezogen auf reines Dihydro-α-ionon) einer farblosen Flüssigkeit erhalten, die 72,3% exo-Isomeres und 20,1% endo-Isomeres des gewünschten Bicyclo-nonans enthält.

### Beispiel 2

### 2-Aethoxy-9-methylen-2,6,6-trimethyl-bicyclo[3.3.1]nonan

480 g (2,21 Mol)) Dihydro-α-ionon, 368 g (2,48 Mol) Orthoameisensäure-triäthylester und 480 ml abs. Aethanol werden unter Rühren während 15 Minuten tropfenweise mit einer Lösung von 34,5 g (0,31 Mol) Bortrifluorid-ätherat in 500 ml Aethanol versetzt und anschliessend während 60 Minuten auf 46-48° Innentemperatur erhitzt. Nach Kühlen des Reaktionsgemisches auf Zimmertemperatur wird mit 600 ml einer 10%igen wässrigen Natriumhydroxid-lösung verrührt. Nach Extrahieren mit Aether und Eindampfen der organischen Phase erhält man 668,7 g einer schwach gelben Flüssigkeit, die im Vakuum flachdestilliert 480,5 g Rohprodukt (Sdp. 45-73°, 0,07 mbar) ergibt. Nach Fraktionierung im Vakuum über eine 20 cm-Widmerkolonne werden 406,2 g (82,7% d.Th.) einer leicht gelblich gefärbten Flüssigkeit erhalten (Sdp. 48-67°, 0,06 mbar), die das gewünschte Bicyclo-nonan in einer Isomerenverteilung von exo:endo = 2:1 enthält.

### Beispiel 3

### 2-Methoxy-9-methylen-2,6,6-trimethyl-bicyclo[3.3.1]nonan

100 g Montmorillonit K-10 (Fluka AG, Buchs, Schweiz) werden mit 450 ml Orthoameisensäure-trimethylester verrührt. Die Suspension wird über eine Nutsche filtriert und abgesaugt. Der Filterrückstand (216,3 g) wird in 300 ml Hexan suspendiert. Zu der auf 0° gekühlten Suspension werden während 5-10 Minuten unter Rühren tropfenweise 53,5 g (0,258 Mol) Dihydro-α-ionon zugegeben. Die Suspension wird anschliessend während 15 Minuten bei 0° gerührt. Nach Filtration über eine Nutsche und Einengen des Filtrates verbleiben 63,7 g einer farblosen Flüssigkeit, die zu ca. 95% aus 6-(3,3-Dimethoxybutyl)-1,5,5-trimethyl-cyclohexen (Dihydro-α-ionon-dimethylketal) besteht.

Das rohe Dimethylketal lässt sich durch Vakuumdestillation über etwas Natriumcarbonat reinigen; Sdp. 61-65°/0,04-0,05 mbar.

14,1 g rohes Dimethylketal werden mit 0,1 g Montmorillonit K-10 versetzt und in einer Destillationspparatur auf 110° erhitzt. Bei dieser Temperatur beginnt Methanol abzudestillieren. Nach ca. 2 Stunden (110-120°) geht kein Methanol mehr über. Nach Kühlen auf 45° wird im Vakuum (0,05-0,06 mbar) fraktioniert. Das Produkt enthält 80% des gewünschten Bicyclo-nonans mit einem Anteil von 55,2% exo- und 24,8% endo-Isomeren.

### Beispiel 4

### 2-Methoxy-9-methylen-2,6,6-trimethyl-bicyclo[3.3.1]nonan

50,8 g rohes Dimethylketal (Reinheit 95%, 0,2 Mol) werden in einer Destillationsapparatur in Gegenwart von Glas-Raschig-ringen auf 320° erhitzt. Bei einer Temperatur von 230° beginnt Methanol abzudestillieren. Nach ca. 7 Stunden ist das Dimethylketal umgesetzt. Das Reaktionsgut wird auf ca. 50° gekühlt und über eine 10 cm-Widmer-Kolonne im Vakuum destilliert. Bei 52-56°/0,05 mbar gehen 34,4 g (81,7% d.Th.) einer farblosen Flüssigkeit über, die zu ca. 93% aus einem Isomerengemisch von 6-(3-Methoxy-butenyl)-1,5,5-trimethyl-cyclohexen [nämlich E- und Z-6-(3-Methoxy-2-butenyl)- sowie 6-(3-Methoxy-3-butenyl)-1,5,5-trimethyl-cyclohexen] besteht.

2,0 g dieses Enoläther-gemisches (9,6 mMol) werden in 3,5 ml Methylenchlorid gelöst und bei 0° mit 2,4 ml einer 10%igen Lösung von Bortrifluorid-ätherat in Methylenchlorid versetzt. Nach 5 Minuten wird über eine Schicht Alox B, Akt. l (JCN Biomedicals, Eschwege, Deutschland) filtriert. Nach Eindampfen des Lösungsmittels werden 1,9 g (95% d.Th.) einer farblosen Flüssigkeit erhalten, die 68,7% exo-Isomeres und 4,6% endo-Isomeres des gewünschten Bicyclo-nonans enthält.

### Beispiel 5

### 2-Aethoxy-9-methylen-2,6,6-trimethyl-bicyclo[3.3.1]nonan

19,4 g Dihydro-α-ionon (Reinheit 93,6%, 0,094 Mol), 25,5 g Orthoameisensäure-triäthylester, 10 ml Aethanol und 5,0 g Montmorillonit KSF (Fluka AG, Buchs, Schweiz) werden während ca. 32 Stunden bei Zimmertemperatur gerührt. Nach Filtration und Eindampfen des Filtrates werden 26,7 g einer gelblichen Flüssigkeit erhalten, die 88% 6-(3,3-Diäthoxybutyl)-1,5,5-trimethyl-cyclohexen (Dihydro-α-ionon-diäthylketal) neben ca. 4,5% Edukt enthalten.

Das rohe Diäthylketal lässt sich durch Flachdestillation im Vakuum über Natriumcarbonat reinigen; Sdp. 67-74°/0,06-0,07 mbar.

Bei der Destillation des rohen Diäthylketals über eine 15 cm-Widmer-Kolonne wird beim Erreichen einer Badtemperatur von ca. 140° Aethanol abgespalten, der bis zu einer Badtemperatur von ca. 160° ausdestilliert. Der Destillationsrückstand wird auf 80-82° abgekühlt und das entstandene Produkt im Vakuum fraktioniert. Bei 82-86°/0,09-0,1 mbar geht eine farblose Flüssigkeit über, die zu 88,5% aus einem Isomerengemisch von 6-(3-Aethoxy-butenyl)-1,5,5-trimethyl-cyclohexen [nämlich E- und Z-6-(3-Aethoxy-2-butenyl)- sowie 6-(3-Aethoxy-3-butenyl)-1,5,5-trimethylcyclohexen] besteht (Ausbeute: 73,0% d.Th. bezogen auf eingesetztes Dihydro-α-ionon).

2,0 g dieses Enoläthergemisches (7,9 mMol) werden in 3 ml Methylenchlorid gelöst und bei 0° mit 2,2 ml einer 10%igen Lösung von Bortrifluorid-ätherat in Methylenchlorid versetzt. Nach 5 Minuten wird über eine Schicht Alox B, Akt. l (s. Beispiel 4) filtriert. Nach Eindampfen des Lösungsmittels werden 1,9 g (95% d.Th.) einer farblosen Flüssigkeit erhalten, die die exo- und endo-Isomeren des gewünschten Bicyclo-nonans in einem Verhältnis von exo:endo = 5:1 enthält.

### Beispiel 6

### Riechstoffkompositionen

In den folgenden Beispielen wird unter Produkt I ein erfindungsgemässes Gemisch Ia:Ib = 2:1, mit R = Aethyl und unter Produkt X wird ein bekanntes Produkt - siehe US-PS 2803662 (Beispiele 3, 5) - verstanden. Im bekannten Fall ist das entsprechende Isomeren-Verhältnis Ia:Ib ca. 1:2.

Der Grund des Auftretens verschiedener Reaktionsprodukte wird darin erblickt, dass im Falle des Standes der Technik von Dihydro-α-ionon ausgegangen, und im vorliegenden Fall von einem Dihydro-α-ionon-di-C₁₋₄-alkylketal oder einen C₁₋₄-Alkylenoläther von Dihydro-α-ionon ausgegangen wird. Im bekannten Fall wird zudem von tieferen - im Beispiel 2 z. B. von -13°C, im vorliegenden Fall von höheren Temperaturen Gebrauch gemacht, wodurch die zu verschiedenen Gemischen führenden Reaktionswege erklärbar sind, siehe auch Helv.Chim.Acta 38, [6], 1955, 1573 seq.

### a) Maiglöckchenbase

| | Gewichtsteile | |
|---|---|---|
| Linalylacetat synth. | 30 | 30 |
| Benzylacetat | 30 | 30 |
| Linalool synth. | 40 | 40 |
| Geranylacetat | 50 | 50 |
| Lyral® (IFF) | 100 | 100 |
| Citronellol extra | 100 | 100 |
| Benzylsalicylat | 100 | 100 |
| Sandela® (Givaudan) | 100 | 100 |
| α-Hexylzimtaldehyd | 200 | 200 |
| Produkt I | 250 | --- |
| Produkt X | --- | 250 |

Der Zusatz des erfindungsgemässen Gemisches I verleiht dieser Komposition dank seiner ambrigen Note einen wesentlich eleganteren, blumigen Charakter, der sich vor allem in den 24-Stunden-Werten der Beurteilung manifestiert und selbst nach 3 Tagen noch deutlich zu erkennen ist. Die Komposition mit "X" wirkt dagegen "rauh". Die Diffusion wird ganz allgemein durch den Zusatz von I im Vergleich zu "X" gesteigert. Dieser unerwartete, vorstärkende Effekt lässt sich über mehrere Tage verfolgen.

### b) Fougèrebase

| | Gewichtsteile | |
|---|---|---|
| Cumarin kristallisiert | 20 | 20 |
| Basilikumöl | 20 | 20 |
| Geraniumöl China | 20 | 20 |
| Baummoosabsolue | 40 | 40 |
| Amylsalicylat | 50 | 50 |
| Benzylsalicylat | 100 | 100 |
| Lavendelöl 40/42% | 350 | 350 |
| Produkt I | 400 | --- |
| Produkt X | --- | 400 |

Der Fougère-akkord mit "X" weist, frisch getaucht, einen eher unangenehmen, aufdringlichen Duft auf, der an die typischen Gerüche von Lavendelöl-Destillationsanlagen erinnert. Dagegen bringt der Zusatz des erfindungsgemässen Gemisches I die sogenannten ländlichen ("agreste") Duftnoten in ausgezeichneter Weise mit den holzigen und moosigen Noten in Einklang und gibt dem Akkord Weiche und Fülle, die selbst über mehrere Tage im Geruchsablauf klar zu erkennen sind.

### c) Tabakbase

| | Gewichtsteile | |
|---|---|---|
| Eugenol, rein | 20 | 20 |
| Cumarin krist. | 30 | 30 |
| Benzoe-resinoid Siam | 50 | 50 |
| 2,3,6,6-Tetramethyl-cyclohex-(2)- | Gemisch 50 | 50 |
| en-carbonsäureäthylester + 2-Aeth- | | |
| yl-6,6-dimethyl-cyclohex(2)-en-ca- | | |
| rbonsäureäthylester | | |
| Phenoxyäthylisobutyrat | 100 | 100 |
| Methylcedrylketon | 150 | 150 |
| Sandela® (Givaudan) | 200 | 200 |
| Product I | 400 | --- |
| Product X | --- | 400 |

In diesem Akkord zeigt der Zusatz des erfindungsgemässen Gemisches I - dies im Gegensatz zum Produkt X - eine unerwartete Süsse im frisch getauchten Zustand, die nach 24 Stunden einer Unterstreichung der holzig-ambrigen und würzigen Note Platz macht. Der Akkord mit "X" wirkt dagegen fettiger und zeigt weniger Charakter. Fülle und Diffusion des Tabak-akkords werden nur durch den Zusatz von I eindeutig unterstrichen und verstärkt.

Aus den obigen Beispielen 6 a) bis c) geht klar hervor, dass sich mit einem Gemisch I, in dem erfindungsgemäss das exo-Isomere überwiegt, Effekte erzielen lassen, die mit einem bekannten Gemisch, in dem das endo-Isomere überwiegt, nicht erzeugen lassen: Die neuen Gemische I sind eben insbesondere "kräftigere" Riechstoffe.

## Patentansprüche (Patentansprüche für folgende(n) Vertragsstaat(en): CH, DE, FR, GB, IT, LI, NL)

1. Gemische von Verbindungen der Formel 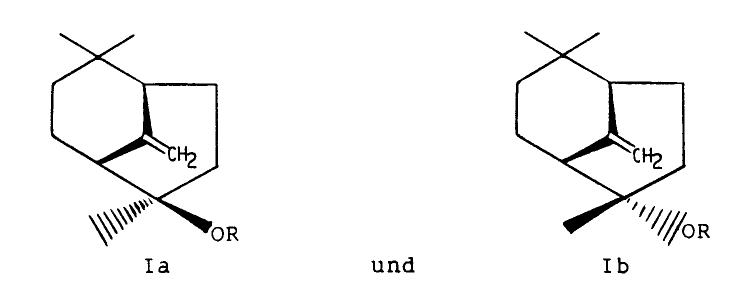 worin R C₁₋₄-Alkyl bedeutet,
im einem Verhältnis von mindestens 1,1:1, insbesondere von 2,2-3,0:1.

2. Gemische gemäss Anspruch 1, worin R Methyl bedeutet.

3. Gemische gemäss Anspruch 2, worin R Aethyl bedeutet.

4. Enoläther der Formel 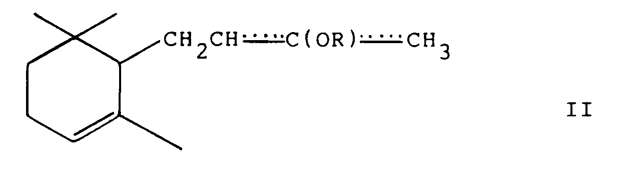 worin R C₁₋₄-Alkyl bedeutet und eine der gestrichelten Linien eine zusätzliche Bindung darstellt.

5. Ketale der Formel 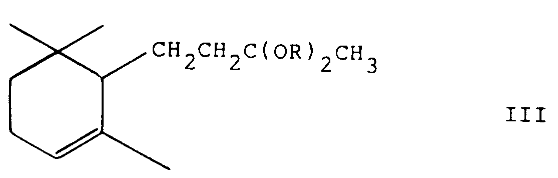 worin R C₁₋₄-Alkyl bedeutet.

6. Riechstoffkomposition, gekennzeichnet durch einen Gehalt an einem Gemisch gemäss einem der Ansprüche 1 bis 3.

7. Verfahren zur Herstellung der Gemische gemäss einem der Ansprüche 1 bis 3, dadurch gekennzeichnet, dass man ein Dihydro-α-ionon-di-C₁₋₄-alkylketal oder einen C₁₋₄-Alkylenoläther von Dihydro-α-ionon mittels einer starken Säure cyclisiert.

8. Verfahren gemäss Anspruch 7, dadurch gekennzeichnet, dass man die starke Säure in katalytischen Mengen verwendet.

9. Verfahren nach Anspruch 8, dadurch gekennzeichnet, dass als starke Säure eine Protonsäure oder eine Lewissäure verwendet wird.

10. Verfahren nach einem der Ansprüche 7-9, dadurch gekennzeichnet, dass man Chlorwasserstoffgas, Schwefelsäure, Phosphorsäure, Trifluoressigsäure, Methansulfonsäure, p-Toluolsulfonsäure, Camphersulfonsäure, BF₃, BF₃·Aet₂O, BF₃·H₃PO₄ oder einen sauren Ionentauscher verwendet.

11. Verfahren nach einem der Ansprüche 7 bis 10, dadurch gekennzeichnet, dass man das Ausgangsmaterial durch Umsetzung von Dihydro-α-ionon mit dem entsprechenden Ketalisierungsmittel bei Temperaturen von ca. 40-50°C in situ gewinnt.

12. Verfahren gemäss Anspruch 11, dadurch gekennzeichnet, dass man als Ketalisierungsmittel eine Verbindung der Formel HC(OR)₃ verwendet.

13. Verfahren nach Anspruch 11 oder 12, dadurch gekennzeichnet, dass man das Ausgangsmaterial unter den Reaktionsbedingungen seiner Herstellung zum Gemisch Ia/Ib cyclisiert.

14. Verwendung der Gemische gemäss einem der Ansprüche 1 bis 3 als Riechstoffe.

## Patentansprüche (Patentansprüche für folgende(n) Vertragsstaat(en): ES)

1. Verfahren zur Herstellung von Gemischen von Verbindungen der Formel 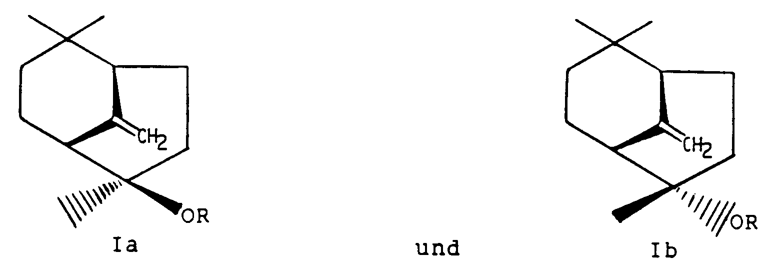 worin R C₁₋₄-Alkyl bedeutet,
im einem Verhältnis von mindestens 1,1:1, insbesondere von 2,2-3,0:1, dadurch gekennzeichnet, dass man ein Dihydro-α-ionon-di-C₁₋₄-alkylketal oder einen C₁₋₄-Alkylenoläther von Dihydro-α-ionon mittels einer starken Säure cyclisiert.

2. Verfahren gemäss Anspruch 1, dadurch gekennzeichnet, dass man die starke Säure in katalytischen Mengen verwendet.

3. Verfahren nach Anspruch 2, dadurch gekennzeichnet, dass als starke Säure eine Protonsäure oder eine Lewissäure verwendet wird.

4. Verfahren nach einem der Ansprüche 1-3, dadurch gekennzeichnet, dass man Chlorwasserstoffgas, Schwefelsäure, Phosphorsäure, Trifluoressigsäure, Methansulfonsäure, p-Toluolsulfonsäure, Camphersulfonsäure, BF₃, BF₃·Aet₂O, BF₃·H₃PO₄ oder einen sauren Ionentauscher verwendet.

5. Verfahren nach einem der Ansprüche 1 bis 4, dadurch gekennzeichnet, dass man das Ausgangsmaterial durch Umsetzung von Dihydro-α-ionon mit dem entsprechenden Ketalisierungsmittel bei Temperaturen von ca. 40-50°C in situ gewinnt.

6. Verfahren gemäss Anspruch 5, dadurch gekennzeichnet, dass man als Ketalisierungsmittel eine Verbindung der Formel HC(OR)₃ verwendet.

7. Verfahren nach Anspruch 5 oder 6, dadurch gekennzeichnet, dass man das Ausgangsmaterial unter den Reaktionsbedingungen seiner Herstellung zum Gemisch Ia/Ib cyclisiert.

8. Riechstoffkompositionen, gekennzeichnet durch einen Gehalt an einem Gemisch von Verbindungen der Formel 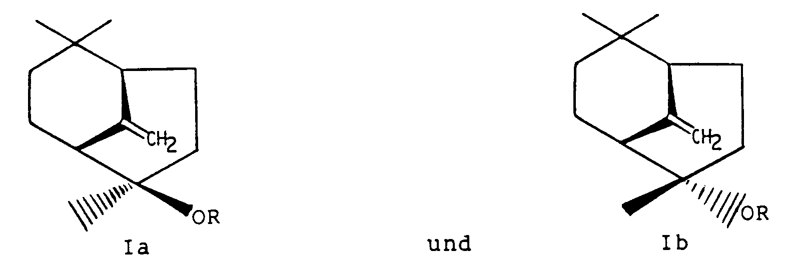 worin R C₁₋₄-Alkyl bedeutet,
im einem Verhältnis von mindestens 1,1:1, insbesondere von 2,2-3,0:1.

9. Verwendung eines Gemisches von Verbindungen der Formel 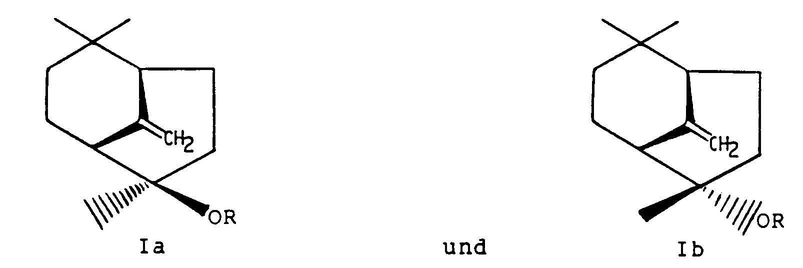 worin R C₁₋₄-Alkyl bedeutet,
im einem Verhältnis von mindestens 1,1:1, insbesondere von 2,2-3,0:1 als Riechstoff.

## Claims (Claims for the following Contracting State(s): CH, DE, FR, GB, IT, LI, NL)

1. Mixtures of compounds of the formulae 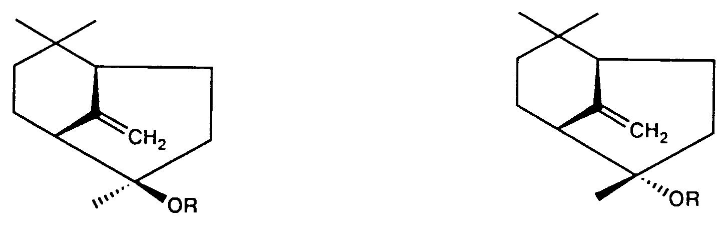 wherein R signifies C₁₋₄-alkyl,
in a ratio of at least 1.1:1, especially of 2.2-3.0:1.

2. Mixtures in accordance with claim 1, wherein R signifies methyl.

3. Mixtures in accordance with claim 1, wherein R signifies ethyl.

4. Enol ethers of the formula 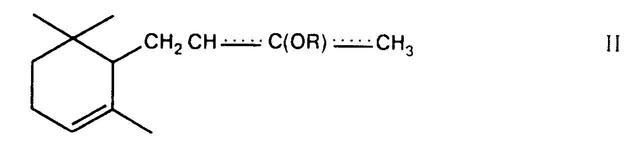 wherein R signifies C₁₋₄-alkyl and one of the dotted lines represents an additional bond.

5. Ketals of the formula 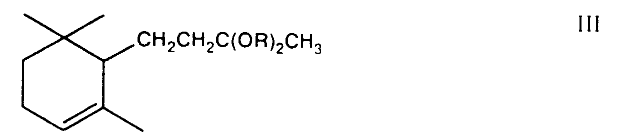 wherein R signifies C₁₋₄-alkyl.

6. An odorant composition, characterized by a content of a mixture in accordance with any one of claims 1 to 3.

7. A process for the manufacture of the mixtures in accordance with any one of claims 1 to 3, characterized by cyclizing a dihydro-α-ionone di-C₁₋₄-alkyl ketal or a C₁₋₄-alkyl enol ether of dihydro-α-ionone by means of a strong acid.

8. A process according to claim 7, characterized in that the strong acid is used in catalytic amounts.

9. A process according to claim 8, characterized in that a protonic acid or a Lewis acid is used as the strong acid.

10. A process according to any one of claims 7-9, characterized in that hydrogen chloride gas, sulphuric acid, phosphoric acid, trifluoroacetic acid, methanesulphonic acid, p-toluenesulphonic acid, camphorsulphonic acid, BF₃, BF₃·Et₂O, BF₃·H₃PO₄ or an acidic ion exchanger is used.

11. A process according to any one of claims 7 to 10, characterized in that the starting material is prepared in situ by reacting dihydro-α-ionone with the corresponding ketalizing agent at temperatures of about 40-50°C.

12. A process according to claim 11, characterized in that a compound of the formula HC(OR)₃ is used as the ketalizing agent.

13. A process according to claim 11 or 12, characterized in that the starting material is cyclized under the reaction conditions of its preparation to the mixture Ia/Ib.

14. The use of the mixtures in accordance with any one of claims 1 to 3 as odorants.

## Claims (Claims for the following Contracting State(s): ES)

1. A process for the manufacture of mixtures of compounds of the formulae 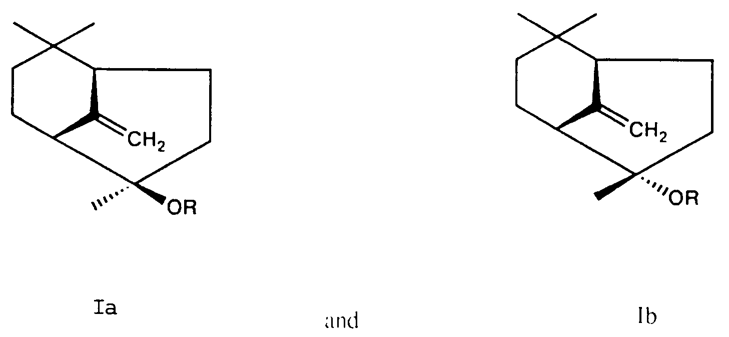 wherein R signifies C₁₋₄-alkyl,
in a ratio of at least 1.1:1, especially of 2.2-3.0:1, characterized by cyclizing a dihydro-α-ionone di-C₁₋₄-alkyl ketal or a C₁₋₄-alkyl enol ether of dihydro-α-ionone by means of a strong acid.

2. A process according to claim 1, characterized in that the strong acid is used in catalytic amounts.

3. A process according to claim 2, characterized in that a protonic acid or a Lewis acid is used as the strong acid.

4. A process according to any one of claims 1-3, characterized in that hydrogen chloride gas, sulphuric acid, phosphoric acid, trifluoroacetic acid, methanesulphonic acid, p-toluenesulphonic acid, camphorsulphonic acid, BF₃, BF₃·Et₂O, BF₃·H₃PO₄ or an acidic ion exchanger is used.

5. A process according to any one of claims 1 to 4, characterized in that the starting material is prepared in situ by reacting dihydro-α-ionone with the corresponding ketalizing agent at temperatures of about 40-50°C.

6. A process according to claim 5, characterized in that a compound of the formula HC(OR)₃ is used as the ketalizing agent.

7. A process according to claim 5 or 6, characterized in that the starting material is cyclized under the reaction conditions of its preparation to the mixture Ia/Ib.

8. Odorant compositions, characterized by a content of a mixture of compounds of the formulae 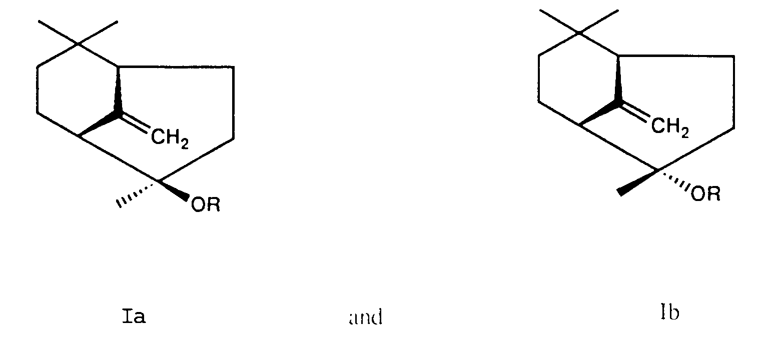 wherein R signifies C₁₋₄-alkyl,
in a ratio of at least 1.1:1, especially of 2.2-3.0:1.

9. The use of a mixture of compound of the formulae 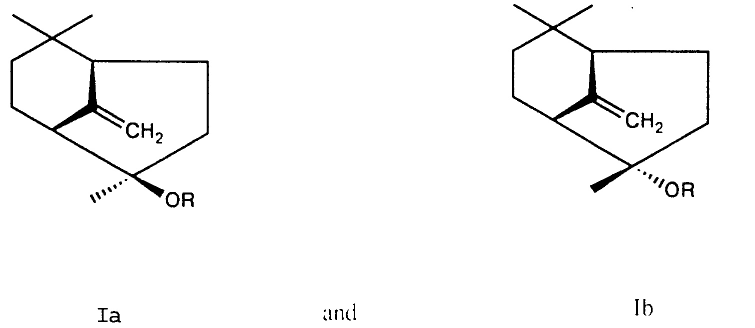 wherein R signifies C₁₋₄-alkyl,
in a ratio of at least 1.1:1, especially of 2.2-3.0:1, as an odorant.

## Revendications (Revendications pour l'(les) Etat(s) contractant(s) suivant(s): CH, DE, FR, GB, IT, LI, NL)

1. Mélanges de composés de formule 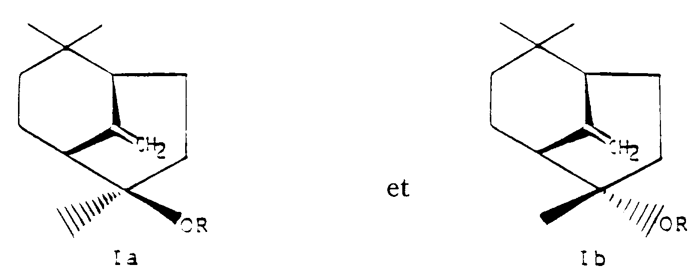 dans lesquelles R représente un groupe alkyle en C1-C4,
dans des proportions relatives d'au moins 1,1 : 1, plus spécialement de 2,2 à 3,0 : 1.

2. Mélanges selon revendication 1, pour lesquels R représente un groupe méthyle.

3. Mélanges selon revendication 1, pour lesquels R représente un groupe éthyle.

4. Ethers d'énols de formule 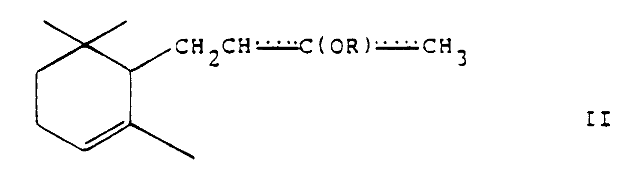 dans laquelle R représente un groupe alkyle en C1-C4 et l'un des traits interrompus représente une liaison supplémentaire.

5. Cétals de formule 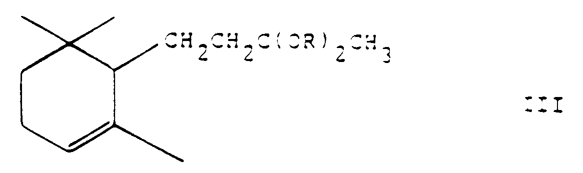 dans laquelle R représente un groupe alkyle en C1-C4.

6. Composition odorante caractérisée en ce qu'elle contient un mélange selon une des revendications 1 à 3.

7. Procédé de préparation des mélanges selon une des revendications 1 à 3, caractérisé en ce que l'on cyclise à l'aide d'un acide fort un di-(alkyle en C1-C4)- cétal de la dihydro-alpha-ionone ou un éther d'alkylénol en C1-C4 de la dihydro-alpha-ionone.

8. Procédé selon revendication 7, caractérisé en ce que l'on utilise l'acide fort en quantité catalytique.

9. Procédé selon revendication 8, caractérisé en ce que l'acide fort consiste en un acide protonique ou un acide de Lewis.

10. Procédé selon une des revendications 7 à 9, caractérisé en ce que l'on utilise le gaz chlorhydrique, l'acide sulfurique, l'acide phosphorique, l'acide trifluoracétique, l'acide méthane-sulfonique, l'acide p-toluène-sulfonique, l'acide camphosulphonique, BF₃, BF₃. Et₂O, BF₃ .H₃po₄ ou un échangeur d'ions acide.

11. Procédé selon une des revendications 7 à 10, caractérisé en ce que l'on forme le produit de départ in situ en faisant réagir la dihydro-alpha-ionone avec l'agent cétalisant correspondant à des températures d'environ 40 à 50°C.

12. Procédé selon revendication 11, caractérisé en ce que l'on utilise en tant qu'agent cétalisant un composé de formule HC(OR)₃.

13. Procédé selon revendication 11 ou 12, caractérisé en ce que l'on cyclise le produit de départ en le mélange Ia/Ib dans les conditions de réaction de sa préparation .

14. Utilisation des mélanges selon une des revendications 1 à 3 en tant que matières odorantes.

## Revendications (Revendications pour l'(les) Etat(s) contractant(s) suivant(s): ES)

1. Procédé de préparation de mélanges de composés de formules 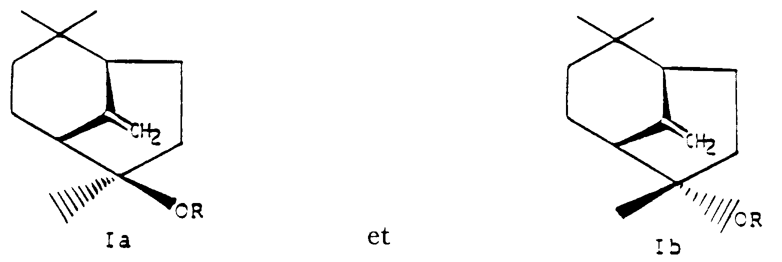 dans lesquelles R représente un groupe alkyle en C1-C4, dans des proportions relatives d'au moins 1,1 : 1, plus spécialement de 2,2 à 3,0 : 1 , caractérisé en ce que l'on cyclise à l'aide d'un acide fort un di - (alkyle en C1-C4)- cétal de la dihydro-alpha-ionone ou un éther d'alkylénol en C1-C4 de la dihydro-alpha-ionone.

2. Procédé selon revendication 1, caractérisé en ce que l'on utilise l'acide fort en quantité catalytique.

3. Procédé selon revendication 2, caractérisé en ce que l'acide fort consiste en un acide protonique ou en un acide de Lewis.

4. Procédé selon revendication1 à 3, caractérisé en ce que l'on utilise du gaz chlorhydrique de l'acide sulfurique, de l'acide phosphorique, de l'acide trifluoroacétique, de l'acide méthane-sulfonique, de l'acide p-toluène-sulfonique, de l'acide campho-sulfonique, BF₃, BF₃. Et₂O, BF₃ .H₃po₄ ou un échangeur d'ions acide.

5. Procédé selon une des revendications 1 à 4, caractérisé en ce que l'on forme le produit de départ in situ par réaction de la dihydro-alpha-ionone avec l'agent cétalisant correspondant à des températures d'environ 40 à 50°C.

6. Procédé selon revendication 5, caractérisé en ce que l'on utilise en tant qu'agent cétalisant un composé de formule HC(OR)₃.

7. Procédé selon revendication 5 ou 6, caractérisé en ce que l'on cyclise le produit de départ en le mélange Ia/Ib dans les conditions de réaction de sa préparation.

8. Compositions odorantes caractérisées en ce qu'elles contiennent un mélange de composés de formules 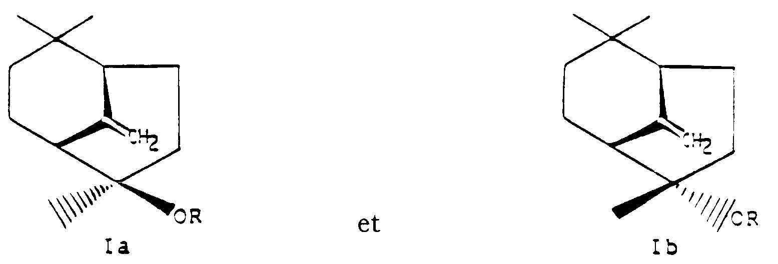 dans lesquelles R représente un groupe alkyle en C1-C4, dans des proportions relatives d'au moins 1,1 : 1, plus spécialement de 2,2 à 3.0 : 1.

9. Utilisation d'un mélange de composés de formules 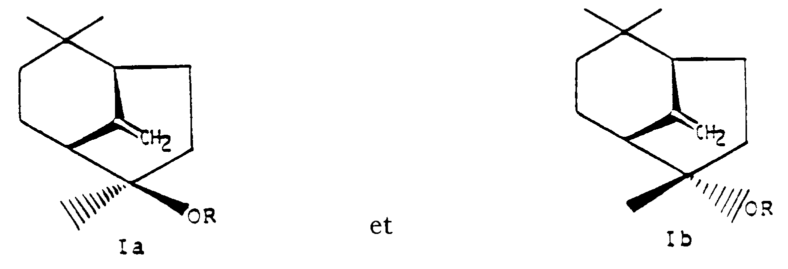 dans laquelle R représente un groupe alkyle en C1-C4, dans des proportions relatives d'au moins 1,1 : 1, plus spécialement de 2,2 à 3,0 : 1, en tant que matière odorante.
